Europäisches Patentamt

European Patent Office

Office européen des brevets

(11) Veröffentlichungsnummer **0 018 471**
**B1**

# EUROPÄISCHE PATENTSCHRIFT

(45) Veröffentlichungstag der Patentschrift:
15.09.82

(51) Int Cl.³ **C 07 D 301/10**, B 01 J 23/66,
B 01 J 23/96

(21) Anmeldenummer **80101012.5**

(22) Anmeldetag **29.02.80**

(54) Verfahren zum Aktivieren oder Reaktivieren von Silber-Träger-Katalysatoren.

(30) Priorität: 26.04.79 DE 2916887

(43) Veröffentlichungstag der Anmeldung:
12.11.80 Patentblatt 80/23

(45) Bekanntmachung des Hinweises auf die Patenterteilung:
15.09.82 Patentblatt 82/37

(84) Benannte Vertragsstaaten:
**BE FR GB IT NL SE**

(56) Entgegenhaltungen:
**DE-A-2 352 608**
**FR-A-2 361 155**
**US-A-3 962 136**

**Die Akte enthält technische Angaben, die nach dem Eingang der Anmeldung eingereicht wurden und die nicht in dieser Patentschrift enthalten sind.**

(73) Patentinhaber: **CHEMISCHE WERKE HÜLS AG,
Postfach 1320, D-4370 Marl 1 (DE)**

(72) Erfinder: **Vangermain, Erwin, Dr., Leverkusener Strasse 1, D-4370 Marl (DE)**
Erfinder: **Mengler, Claus-Dieter, Dr., Langehegge 147, D-4370 Marl (DE)**

## Verfahren zum Aktivieren oder Reaktivieren von Silber-Träger-Katalysatoren

Zur Herstellung von Ethylenoxid durch Oxidation von Ethylen mit Sauerstoff oder Sauerstoff enthaltenden Gasen werden Silberkatalysatoren eingesetzt. Es ist auch bekannt, derartige Katalysatoren mit sogenannten Promotoren zu versetzen, wobei insbesondere die Erdalkalimetallverbindungen, vorzugsweise Bariumverbindungen und/oder Alkalimetallverbindungen, insbesondere die der sogenannten schweren Alkalimetalle, Rubidium und/oder Cäsium, verwendet werden (DE-OS 2 300 512, 2 733 688, 2 442 568). Die Promotoren werden üblicherweise gleichzeitig mit dem Silber oder auch nach dem Silberaufzug auf den Träger bei der Herstellung des Katalysators aufgebracht.

Es ist auch bekannt, daß Silberkatalysatoren im Laufe der Zeit an Selektivität verlieren und nach einigen Jahren Gebrauch durch neuen Katalysator ersetzt werden müssen. Der Austausch eines in seiner Leistungsfähigkeit abgefallenen Katalysators durch einen neuen in großtechnischen Anlagen, ist zeitraubend und arbeitsintensiv; er verursacht außerdem Produktionsausfall und hohe Kosten.

Es ist auch bekannt, die Leistungsfähigkeit eines Silberkatalysators zu verbessern bzw. einen benutzten Katalysator zu reaktivieren (DE-OS 2 746 976, DE-AS 2 519 599).

Dies geschieht z. B. in Großanlagen durch Fluten des mit dem Katalysator gefüllten Reaktors mit der Lösung der Promotormetallverbindung. Nach dem Abtrennen des überschüssigen Lösungsmittels wird durch Aufheizen und Durchblasen des Katalysatorbettes mit einem Inertgas das Restlösungsmittel entfernt. Diese Arbeitsweise erfordert das Anwenden von Lösungsmitteln und ebenfalls eine Produktionsunterbrechung.

Die gestellte Aufgabe besteht nun darin, durch eine einfache Behandlungsmethode Katalysatoren in ihrer Leistungsfähigkeit zu verbessern, ihre Leistungsfähigkeit möglichst lange zu erhalten und den Austausch gegen einen neuen Katalysator zu vermeiden bzw. möglichst weit hinauszuschieben.

Die Lösung dieser Aufgabe gelingt, wenn man das Reaktionsgasgemisch (Ethylen plus $O_2$ oder $O_2$ enthaltende Gase) durch eine unmittelbar vor der Katalysatorzone angeordnete Schicht aus grobkörnigen Promotormetallverbindungen aus der Gruppe von Oxiden, Peroxiden, Hydroxiden, Nitriten, Nitraten, Carbonaten, Acetaten, Oxalaten, Tartraten, Stearaten, Dodecanaten des Bariums und/oder des Rubidiums und/oder des Cäsiums oder aus einem mit diesem Promotormetallverbindungen getränkten silberfreien Trägermaterial leitet.

Aus der DE-A-2 353 608 ist ein Verfahren bekannt, wobei zum Moderieren als Halogenverbindungen feste Chloride eingesetzt werden sollen. Moderatoren geben das Halogen laufend in die Gasphase ab,wobei sowohl unterdosieren als auch Überdosieren vermieden werden muß. Das Dotieren mit bestimmten Promotoren aus der Gruppe der schweren Alkali- und der Erdalkalimetalle nach dem Verfahren der Erfindung kann aus dieser Literaturstelle jedoch nicht nahegelegt werden, da Promotormetallverbindungen nach dem Stande der Technik in die Silbermatrix des Katalysators eingebettet werden und vermieden werden muß, daß eine Überdosierung an Promotormetallen auftritt.

Es war nicht zu erwarten, daß ein Aktivieren bzw. Reaktivieren des Katalysators erreicht werden kann.

Die Arbeitsweise ist sowohl anwendbar auf Silberkatalysatoren, die bereits die Promotoren enthalten, um sie in ihrer Leistungsfähigkeit zu erhalten, als auch auf solche Katalysatoren, die solche Promotoren nicht enthalten, um sie in ihrer Leistungsfähigkeit zu verbessern.

Die Promotormetallverbindungen werden in grobkörniger Form verwendet, insbesondere verwendet man eine Trägerschicht, die dem Katalysatorträger entspricht, der mit Lösungen der Promotormetallverbindungen getränkt worden war.

Diese Schicht kann unmittelbar in die einzelnen Reaktionsrohre am Anfang der Katalysatorzone eingebracht werden, d. h. am Einlaß des Reaktionsgases. Es ist dabei auch möglich, die Schicht so zu gestalten, daß sie leicht ausgewechselt werden kann. In einer besonderen Form ist es jedoch auch möglich, diese Schicht in einem besonderen Behälter anzubringen, der in dem Gasstrom des Reaktionsgases angeordnet ist, wobei dieser Behälter möglichst nahe bzw. unmittelbar vor dem Eingang der Katalysatorzone liegt. In einer weiteren Ausgestaltung kann der Behälter auch in einen Teilstrom des Reaktionsgases geschaltet werden, was den Vorteil besitzt, daß dieser Teilstrom nur zeitweise betrieben werden muß. Vorteilhaft wird man die Schicht in den Reaktionsrohren bzw. in den gesonderten Behältern derart gestalten, daß man zunächst eine Schicht reinen Trägermaterials, dann eine Schicht der Promotormetallverbindungen bzw. der mit Promotormetallverbindungen getränkten Trägerschicht folgen läßt und schließlich darüber eine weitere Trägerschicht anbringt.

Eine solche Anwendung hat den Vorteil, daß das Reaktionsgas während des Durchganges in seiner Temperatur verändert werden kann. Behälter und Reaktionsrohr sind oder können mit entsprechenden Heiz- und Regeleinrichtungen ausgerüstet werden.

Der Anteil der einzelnen Promotormetallverbindungen wird in ihrer Menge auf das gewünschte Verhältnis eingestellt. Die Schichthöhe des mit Promotormetallverbindungen getränkten Trägers in den Reaktionsrohren beträgt im allgemeinen 1 bis 20, insbesondere 5 bis 10% der Rohrlänge.

Geeignete Promotormetallverbindungen sind Bariumverbindungen, wie Barium-oxid, -peroxid, -hydroxid, -nitrit, -nitrat, -carbonat, -acetat, -oxalat, -tartrat, -stearat, -dodecanat und/oder Verbindungen des Rubidiums und/oder Cäsiums, wie Oxide, Peroxide, Hydroxide, Nitrite, Nitrate, Carbonate, Acetate, Oxalate, Tartrate, Stearate, Dodecanate.

Die folgenden Beispiele erläutern das Verfahren der Erfindung.

Der Versuchsreaktor besteht aus einem Reaktionsrohr von 6000 mm Länge und 26 mm Durchmesser aus rostfreiem Stahl. Es wurde jeweils ein Rohr mit einem in folgenden Beispielen näher beschriebenen Katalysator gefüllt. Ein zweites Rohr wurde mit jeweils dem gleichen Katalysator gefüllt und zusätzlich an der Gaseinlaßstelle eine Schicht angebracht, die in Richtung des Gasstromes bestand aus 400 mm des unbehandelten Trägermaterials, 450 mm eines mit Promotormetallen beschichteten Trägermaterials und 30 mm unbehandelten Trägermaterials. Die Rohre waren jeweils von einem Mantel umgeben, der Wasser bzw. Wasserdampf zum Abführen der Reaktionswärme enthielt. Die Katalysatoren enthielten jeweils 19.5 Gew.-% Silber. Als Trägermaterial wurde ein Aluminiumoxidträger verwendet, der etwa 12 Gew.-% Siliciumdioxid enthielt. Die Reaktionsrohre wurden gleichzeitig mit der gleichen Gasmischung betrieben. Diese Gasmischung hatte folgende Zusammensetzung:

25 Vol.-% $C_2H_4$
49 Vol.-% $CH_4$
6,8 Vol.-% $O_2$
4,5 Vol.-% $CO_2$
0,2 Vol.-% $C_2H_6$
Rest        Ar $+ N_2$

## Beispiel 1

Es wurden 2,4 kg des beschriebenen Silberkatalysators eingesetzt, der keine zusätzlichen Promotoren enthielt. Die imprägnierte Trägerschicht im zweiten Reaktionsrohr enthielt 200 g Trägermaterial und 1,1 g Kalium, 1,6 g Barium und 0,34 g Cäsium, die in Form einer mit Kaliumcarbonat alkalisch gestellten wäßrigen Lösung von Bariumperoxid und Cäsiumnitrat aufgebracht worden waren.

Die Gasbelastung betrug in beiden Fällen nach Beendigung der Einlaufphase 9,26 $Nm^3$/l Katalysator bei 19,8 bar Überdruck, entsprechend einer Gesamtgasbelastung von 25 $Nm^3$/h. Der Chlorgehalt im Kreisgas wurde mit Hilfe von 1,2-Dichlorethan bei 7 bis 8 mg Cl/$Nm^3$ gehalten. Die Dampfraumtemperatur wurde so reguliert, daß sich eine Ethylenoxidkonzentration von durchschnittlich 1,6 Vol.-% im Reaktorausgang einstellte.

|  | Anfang | | nach 6 Wochen Betriebszeit | |
|---|---|---|---|---|
|  | Selektiv. Mol-% | Temp. °C | Selektiv. Mol-% | Temp. °C |
| Vergleich | 74,0 | 254 | 71,6 | 271 |
| Erfindung | 74,3 | 252 | 74,8 | 251 |

## Beispiel 2

Es wurde als Vergleichskatalysator ein solcher verwendet, der bereits 472 ppm Barium enthielt. Unter sonst gleichen Bedingungen wurden folgende Ergebnisse erhalten:

|  | Anfang | | nach 6 Wochen Betriebszeit | |
|---|---|---|---|---|
|  | Selektiv. Mol-% | Temp. °C | Selektiv. Mol-% | Temp. °C |
| Vergleich | 75,7 | 249 | 73,5 | 267 |
| Erfindung | 75,8 | 249 | 76,7 | 247 |

### Beispiel 3

Es wurde als Vergleichskatalysator ein solcher verwendet, der bereits 481 ppm Barium und 91 ppm Cäsium enthielt. Unter sonst gleichen Bedingungen wurde folgende Ergebnisse erzielt:

| | Anfang | | nach 6 Wochen Betriebszeit | |
|---|---|---|---|---|
| | Selektiv. Mol-% | Temp. °C | Selektiv. Mol-% | Temp. °C |
| Vergleich | 80,4 | 245 | 77,5 | 265 |
| Erfindung | 80,3 | 245 | 80,3 | 245 |

### Beispiel 4

Als Vergleichskatalysator wurde wie im Beispiel 1 ein reiner Silberkatalysator eingesetzt. Erfindungsgemäß enthielt die Trägerschicht anstelle von Cäsium 0,29 g Rudidium. Folgende Ergebnisse wurden erhalten:

| | Anfang | | nach 6 Wochen Betriebszeit | |
|---|---|---|---|---|
| | Selektiv. Mol-% | Temp. °C | Selektiv. Mol-% | Temp. °C |
| Vergleich | 74,0 | 254 | 71,6 | 271 |
| Erfindung | 74,2 | 252 | 73,7 | 255 |

**Patentansprüche**

1. Verfahren zum Aktivieren oder Reaktivieren von Silber-Träger-Katalysatoren für die Herstellung von Ethylenoxid durch Oxidation von Ethylen mit Sauerstoff oder Sauerstoff enthaltenden Gasen mit Hilfe von an sich bekannten Promotormetallen, dadurch gekennzeichnet, daß man das Reaktionsgasgemisch (Ethylen plus $O_2$ oder $O_2$ enthaltende Gase) durch eine unmittelbar vor der Katalysatorzone angeordnete Schicht aus grobkörnigen Promotormetallverbindungen aus der Gruppe von Oxiden, Peroxiden, Hydroxiden, Nitriten, Nitraten, Carbonaten, Acetaten, Oxalaten, Tartraten, Stearaten, Dodecanaten des Bariums und/oder des Rubidiums und/oder des Cäsiums oder aus einem mit diesem Promotormetallverbindungen getränkten silberfreien Trägermaterial leitet.

2. Verfahren nach Anspruch 1, dadurch gekennzeichnet, daß die Schicht im Reaktionsrohr in einer Schichthöhe von 1 bis 20% der Länge des Reaktionsrohres an der Eintrittsstelle des Reaktionsgases angeordnet ist.

3. Verfahren nach Anspruch 1, dadurch gekennzeichnet, daß die Schicht in einem gesonderten Behälter unmittelbar vor der Katalysatorzone angeordnet ist.

**Claims**

1. A process for activating or regenerating a supported silver catalyst for use in the manufacture of ethylene oxide by oxidation of ethylene with oxygen or an oxygen-containing gas with the aid of a known promoter metal, characterised in that the gaseous reaction mixture (ethylene plus $O_2$ or $O_2$-containing gas) is passed through a layer which is arranged immediately before the catalyst zone and is formed of one or more coarsely particulate promoter metal compounds selected from the oxides, peroxides, hydroxides, nitrites, nitrates, carbonates, acetates, oxalates, tartrates, stearates and dodecanates of barium, rubidium and caesium or of a coarsely particulate silverfree carrier material composited with one or more of these promoter metal compounds.

2. A process according to claim 1, characterised in that the layer is arranged in the reaction tube at

4

the point of entry of the reaction gas and has a thickness of from 1 to 20% of the length of the reaction tube.

3. A process according to claim 1, characterised in that the layer is arranged in a special container immediately before the catalyst zone.

**Revendications**

1. Procédé d'activation ou de réactivation de catalyseurs à l'argent sur support pour la fabrication d'oxyde d'éthylène par oxydation d'éthylène au moyen d'oxygène ou de gaz contenant de l'oxygène à l'aide de métaux activants en eux-mêmes connus, caractérisé par le fait que l'on fait passer le mélange gazeux de réaction (éthylène plus $O_2$ ou gaz contenant $O_2$) à travers une couche, disposée immédiatement avant la zone de catalyseur, formée de composés métalliques activants pris dans le groupe des oxydes, peroxydes, hydroxydes, nitrites, nitrates, carbonates, acétates, oxalates, tartrates, stéarates, dodécanates de baryum et/ou de rubidium et/ou de césium ou formée d'une matière support exempte d'argent et imprégnée de ces composés métalliques activants.

2. Procédé selon la revendication 1, caractérisé par le fait que la couche est disposée dans le tube de réaction en une hauteur de couche de 1 à 20% de la longueur du tube de réaction, au point d'entrée du gaz de réaction.

3. Procédé selon la revendication 1, caractérisé par le fait que le couche est disposée dans un récipient séparé, immédiatement avant la zone de catalyseur.